# EUROPEAN PATENT SPECIFICATION

(11) **EP 1 028 842 B1**
(45) Date of publication and mention of the grant of the patent: **04.09.2002**
(21) Application number: 98956550.2
(22) Date of filing: 04.11.1998
(51) Int. Cl.: B29C 55/18

(54) **TEXTURED, DIMENSIONALLY STABLE MICROPOROUS FILM AND METHOD OF MAKING SAME**
TEXTURIERTE, FORMBESTÄNDIGE, MIKROPORÖSE FOLIE UND VERFAHREN ZUR HERSTELLUNG
FILM MICROPOREUX TEXTURE STABLE DIMENSIONNELLEMENT ET PROCEDE DE FABRICATION DE CELUI-CI

(30) Priority: 04.11.1997 US 64185 P; 25.09.1998 US 160652
(43) Date of publication of application: 23.08.2000
(73) Proprietor: Tredegar Film Products Corporation, Richmond, VA 23225 (US)
(72) Inventor: DIPOTO, James, Arlington, TX 76012 (US); HEDDEN, Chad, M., Terre Haute, IN 47802 (US)
(74) Representative: Baverstock, Michael George Douglas
(86) International application number: US9823455
(87) International publication number: WO99022930

(56) References cited:
- EP-A- 0 227 481
- EP-A- 0 352 802
- GB-A- 2 151 538
- US-A- 4 116 892

## Description

### Background

The present invention is in the general field of microporous films. The invention particularly relates to textured microporous films which are dimensionally stable at elevated temperatures and to a method of making such films.

The microporosity of a film is typically expressed in terms of moisture vapor transmission rate or MVTR. This is also commonly referred to as the breathability of the film. One method to determine the MVTR of a film is to use the test procedures as given in ASTM E96-93.

Readily envisioned uses of the present invention include a liquid impervious layer in such products as disposable absorbent articles including disposable garments for sanitary purposes such as catamenial pads, diapers, incontinent articles or for hospital pads, surgical dressings, beds and for other products such as sleeping bag liners and the like.

Absorptive articles such as diapers, catamenial products, surgical drapes and the like are designed to receive and retain a liquid within an absorbent core. The absorptive article contains a cover or backsheet on the exterior of these articles which prevents the absorbed liquid from leaking or striking through the absorbent core. The liquid-proofbacksheet significantly reduces self-drying of the absorptive article by any evaporation of the fluid held in the core. The exterior liquid-proof backsheet can contribute in making the absorptive article hot, clammy and ultimately uncomfortable to wear. Therefore, it would be an advantage to have a breathable material which permits exchange of vapors but retains fluid as a liquid-proof backsheet.

One type of breathable film is a microporous film, which has a plurality of micropores interconnected through tortuous paths extending from one exterior surface of the film to the other exterior surface of the film. This type of a microporous film can be formed by preparing and drawing a film containing at least one type of filler material. The filler can be removed from the film, allowed to remain intact within the film or be crushed under pressure to provide pores in the film. The filler particles can also be caused to separate from the thermoplastic polymers during a stretching process to form interconnecting micropores.

Various patents which show the general principle of using a filler material in a plastic material to produce a microporous film include Aoyama et al. U.S. Patent Nos. 4,841,124 and 4,921,653; Nishizawa et al. U.S. Patent No. 4,626,252; Sugimoto U.S. Patent No. 4,472,328; Schwarz U.S. Patent Nos. 4,833, 172, 4,116,892, 4,289,832, 4,153,571, and 4,091,164. Still other patents which teach modifying inorganic fillers using additional ingredients to produce a microporous film include Hwang U.S. Patent No. 4,824,718; Toyoda et al., U.S. Patent No. 4,418,112; Takashi et al. U.S. Patent No. 4,319,950; Suzuki U.S. Patent No. 3,969,562; Ikeda et al. U.S. Reissue Patent No. 28,608; Ikeda et al. U.S. Patent Nos. 3,738,904 and 3,903,234; Elton et al. U.S. Patent Nos. 3,870,593 and 3,844,865; Ita et al. U.S. Patent No. 4,705,812; Hogue U.S. Patent No. 4,350, 656; Antoon, Jr. et al. U.S. Patent Nos. 5,008,296 and 4,879,078; Okuyama et al. U.S. Patent Nos. 4,704,238 and 4,585,604; Doi et al. U.S. Patent Nos. 4,335,193,4,331,622 and 4,210,709; Kaneko et al. U.S. Patent No. 5,445,862; McCormick PCT Application WO95/16562; Sheth U.S. Patent No. 4,929,303 and 4,777,073; Sheth et al. U.S. Patent No. 5,055,338; Cancio et al. U.S. Patent Nos. 5,055,338, 4,380,564 and 4,298,647; Gurewitz U.S. Patent No. 5,364,695; Seiss et al. U.S. Patent No. 4,716,197; Sheth U.S. Patent No. 4,777,073; and Exxon PCT Application WO98/04397.

While it would be desirable to use high amounts of filler material in a textured microporous film, various difficulties occur when using the currently known film forming technologies discussed above.

In order to improve the physical properties of thermoplastic films, various methods have been used to stretch and orient the films. One method involved passing the film through sets of rolls operating at different speeds. Another method involved passing the film through intermeshing gear rolls which stretch the film. Various patents which show the general principle using intermeshing gears to stretch a film include: Schwarz U.S. Patent Nos. 4,116,892; 4,144,008; 4,153,751; 4,251,585; 4,223,059; 4,285,100; 4,289,832; 4,368,565 and 4,438,167. While intermeshing gear orienting processes have been used in the past to stretch film, until the present invention, no one had successfully uniaxially or biaxially stretched a film containing a high amount of filler, to provide an improved textured, soft, flexible and dimensionally stable microporous film.

In the past, the filler-containing microporous films were not acceptably soft and flexible and were not candidates for use in disposable products. In the past, microporous films were embossed to enhance the film's texture, softness and flexibility. However, microporous films having a high filler content are difficult to emboss without significantly reducing the physical properties of this film. Melt embossing, or embossing prior to orientation, causes the film to have thinner and thicker areas prior to orientation. The thin areas are more likely to develop pinholes and tears during orientation or during later processing. Further, reheat embossing of microporous films generally results in inferior breathability. The presence of high amounts of filler in a microporous film increases the thermal conductivity of the film. The increase in thermal conductivity results in the polymer surrounding the filler particles relaxing during the embossing step. The relaxation of the polymer causes the polymer to flow into the voids in the film and reduces the porosity of the film. Until the present invention, this phenomenon has caused microporous films to have a significantly reduced breathability after embossing.

The presence of a filler in the film formulation presented additional limitations to the film forming process. The presence of high amounts of filler in a film changes the thermal conductivity of the film. Until the present invention, the heat generated during the extrusion and film forming processes have made it virtually impossible to achieve a textured dimensionally stable, microporous film without causing damage to the micropores in the film.

When a high filler content formulation is to be further processed or converted into an end product, the heat characteristics of the filler-containing film cause additional concerns. Since the filler quickly heats, subsequent application of heat, hot glue, or adhesives to the film causes shrinkage, puckering, and/or holes in the microporous film.

When prior art films are used in disposable products such as diapers or catamenials, there is usually an adhesive strip that is used to secure the disposable article. Frequently, due to the presence of filler particles on the surface of the generally flat microporous films, the adhesive bonds to the disposable product so that it is difficult to peel the adhesive strip from the film, or to peel the film from an article to which it is attached. Such a high peeling force often results in the tearing of the film or the article to which it is attached, which is not acceptable to the consumer. Until the present invention, it was virtually impossible to reduce adhesive peeling forces of microporous films.

It is well known in the art that oriented films have reduced dimensional stability at elevated temperatures. This lack of dimensional stability at elevated temperatures is exacerbated by the increased thermal conductivity of microporous films. This creates problems during further processing and in shipping. For example, a trailer truck can reach 60°C (140°F), which can cause the film to shrink. This is not acceptable because the shrinkage will cause the plies of film on the roll to stick together, preventing unwinding of the film. Until the present invention, microporous films had poor dimensional stability at elevated temperatures.

The presence of a high amount of filler in a film has, in the past, limited the physical properties of the film, including the ability of such a film to be stretched. Since the filler material is inelastic, the filler-containing film only stretches a limited amount before reaching its yield point. Once the filler-containing film passed its yield point, the film quickly reached its break point. This narrow window between the yield point and the break point prevented the film from having the capability of being further stretched in subsequent processing or converting steps. Any further elongation or processing of the film by an end user in converting the film into an absorptive disposable product caused degradation of the physical properties of the film. Such prior art films were rather stiff and had paper-like properties. These prior art films did not have the soft and flexible characteristics of the film of the present invention.

While various formulations have been used in the past to produce thermoplastic films, no one has produced a textured dimensionally stable microporous film having a high percent of filler material and a polyolefin blend comprising high density polyethylene, or other heat resistant polymer, and linear low density polyethylene. Optionally the film formula can include low density polyethylene and processing aids.

It is therefore the primary object of the present invention to provide a textured, dimensionally stable microporous film or sheet useful in disposable articles and a process for the preparation of such film. The invention's added feature of dimensional stability provides even greater utility to such microporous film.

It is therefore another object of the present invention to provide an improved method for forming a dimensionally stable microporous film which can be further stretched during converting and still meet the end use requirements for the film.

### SUMMARY

The present invention provides a textured microporous film which is vapor permeable, liquid impermeable and is dimensionally stable at elevated temperatures. For the first time, it is disclosed herein a method for making textured, dimensionally stable, microporous film.

According to the present invention, the film formulation provides a balance of high amounts of a filler which a polyolefin blend of linear low density polyethylene and high density polyethylene or other heat resistant polymer and in certain embodiments, low density polyethylene, to provide a film with the desired physical properties. In one embodiment, heat resistant polymers can be incorporated into the blend to create a blend that has a primary melting point, when evaluated by differential scanning calorimetry (DSC), of equal or greater than 120°C; however, blends with a primary melting point of less than 120°C can also be used. Heat resistant polymers can include other thermoplastic or elastomeric polymers with a melting point higher than 120°C. The primary melting point is defined as the peak with the largest area or endothermic energy flux. Examples of heat resistant polymers include, but are not limited to, polypropylenes, rubber modified high density polyethylene, linear medium density polyethylene, ethylene-propylene copolymers, and styrene containing block copolymers.

Among the advantages obtained from the above-described film forming process is that the film is easily oriented to yield the desired controllable permeability without pinholing. The improved moisture vapor transmission properties of the film result from a combination of factors. The first factor is that the film formulation comprises a high percentage of filler material. The second factor is the intermeshing gear orientation of the film where a balanced orientation is achieved in the film. The third factor is the annealing of the oriented film where the temperature and tension in the annealing means sets or cures the micropores in the film. Optionally, the fourth factor is the reheating and embossing of the annealed film where the embossing temperature and the tension between the reheating rolls and engraved rolls provide softness to the film without decreasing the desirable moisture vapor transmission properties.

Another advantage obtained from the above-described film forming process is the residual elongation property of the film. The residual elongation enables the film to be further stretched during converting of the film into an end use product. The residual elongation property, i.e., the difference between the elongation-at-break of the film and the elongation-at-yield of the film, is related to the intermeshing gear orientation of the film in the machine and transverse directions and to the depth of engagement used in the intermeshing gear orientation. The residual elongation property is also dependent on the film formulation. The ability of a film to be oriented depends on the polymer blend used in the film formulation. The ability of the film to be oriented effects how much the film must be elongated in order to have the desired moisture vapor transmission properties. An advantage of the film formulation of the present invention is the low elongation required to achieve the desired vapor transmission rates.

Yet another advantage is that the film has good heat resistance despite having a high filler content. The heat resistance of the film prevents damage to the film from occurring during further applications of heat to the film. In particular, the heat resistance of the film prevents the formation of holes in the film when a hot melt adhesive material is applied to the film during converting of the film into an end use product.

Yet another advantage is that the film has dimensional stability at elevated temperatures and does not readily shrink. Such dimensional stability is related to the intermeshing gear orientation and film formulation. Further dimensional stability is provided to the film during the annealing process by controlling the tension between the rolls, the temperature of the annealing rolls, and the length of time in which the film is in contact with the annealing rolls. The dimensional stability of the film is also increased by controlling the embossing preheat temperature and the tension between the preheat rolls and engraved rolls of the embossing means.

Still another advantage of the film of the present invention is the impact strength of the film. The impact strength of the film is improved by balancing the machine and transverse direction orientation.

Yet another advantage is the production of a film having a desired texture which improves the aesthetics of the film and which decreases adhesive peeling forces to the film. The embossing of the film reduces the contact surface area between an adhesive and in the microporous film. A decrease in the peeling force is an advantage when the film is used in disposable products such as diapers and catamenials. The microporous film of the present invention allows the fastening tape to be removed or the film to be removed from the article to which it is attached without tearing the film.

### BRIEF DESCRIPTION OF THE DRAWINGS

- Fig. 1: is a schematic illustration of a system for producing a textured, dimensionally stable microporous film.
- Fig. 2: is a graph showing the moisture vapor transmission rate for various high filler content films.

### DETAILED DESCRIPTION

The present invention is a soft, textured, dimensionally stable, microporous film and a method for making the film. The film is formed using a combination of a high density polyethylene, or other heat resistant polymers, with a linear low density polyethylene. The film also contains a filler that creates the micropores when the film is subjected to stretching forces. In one embodiment, the microporous film is annealed to provide a greater dimensional stability to the film when the film is subjected to elevated temperatures. In yet another embodiment, the film is embossed.

The balance between the high density polyethylene (or other heat resistant polymer) and linear low density polyethylene, along with the filler provides a film having desired moisture vapor transmission rate properties, along with desired impact strength, dimensional stability (resistance to shrink), and elongation properties. Increasing the amount of high density polyethylene increases the moisture vapor transmission rate and the heat resistance of the film due to the higher crystallinity level. However, amounts of over about 20% of high density polyethylene affect the modulus, stiffness and noise characteristics of the film resulting in reduced consumer appeal. The linear low density polyethylene material allows the film to have the soft tactile qualities needed in disposable products. The linear low density polyethylene also allows the film to be easily stretched due to the extensibility of this component. The presence of linear low density polyethylene in the film formulation also increases the elongation properties of the film.

The film of the present invention is formed using a polyolefin film formulation comprising about 45 to about 65%, preferably, about 50 to about 55%, of a filler material, about 25 to about 55% linear low density polyethylene, about 0 to about 20% high density polyethylene or other heat resistant polymers, about 0 to about 5% low density polyethylene, about 0 to about 3% processing aids, and about 0 to about 10% pigmenting agents, such as whiteners or other coloring agents. Preferably the thermoplastic film formulation comprises about 50 to about 56% calcium carbonate filler, about 30% to about 40% linear low density polyethylene, about 5% to about 10% high density polyethylene or other heat resistant polymer, about 2% processing aid comprising a Viton® fluorocarbon in a low density polyethylene, and about 2% of a white concentrate which comprises a titanium dioxide in a low density polyethylene.

The film in the present invention has very good heat resistance. The polyolefin blend of the present invention combines a higher melting point material (such as high density polyethylene having a melting point of about 130°C) with a material having a somewhat lower melting point temperature (such as linear low density polyethylene having a melting point about 122°C). The heat resistance of the film is surprising since the film has a large amount of a filler. In the past, various hot melt adhesives tended to cause puckering, shrinking, or holes when applied to a microporous film. However, when the high filler content formulation used in the present invention is annealed, there is no damage to the film such as puckering, shrinking, or holes at the glue spots.

The breathability, or MVTR of the film, is affected by the amount and the particle size of the filler and by the relationship between the types of resins used in the film. The percentage of filler that can be present in the film depends, in part, on the size of the filler particles. Filler particles of a certain predetermined size range have defined ranges of circumference (or surface area) and volume. Both the circumference and volume of the filler particles affect the moisture vapor transmission rate of a film. The circumference and the volume of the filler particles cause certain size holes and pathways to be formed in the film. Filler particles having a size of about 12.5 microns or larger, when used with the resins specified above, cause the resulting film to have pinholes through which liquids may leak. It has been found that filler particles have an average size of about 0.5 to 5 microns and a preferred median size of about 1 to about 3 microns work particularly well to produce a film with the desired level of breathability.

Various inorganic filler materials may be used including, but not limited to, calcium carbonate, talc, clay, kaolin, silica, diatomaceous earth, magnesium carbonate, barium carbonate, barium sulfate, calcium sulfate, magnesium sulfate, magnesium hydroxide, aluminum hydroxide, magnesium oxide, calcium oxide, zinc oxide, titanium oxide, alumina, mica, glass powder and the like. These fillers may be used separately or in combination. A particularity suitable filler material is calcium carbonate. The calcium carbonate can be at least partially coated with stearic acid or other stearate compounds which allow the calcium carbonate to have a more even dispersion throughout the resin composition.

The breathability of a film having about 38% filler, by weight, based on the weight of the film, is relatively low, while a film having about 53 to 55% filler has a high MVTR. While additional filler, greater than about 53 to about 55%, can be added to the film, there is no substantial increase in the moisture vapor transmission rate. Levels of filler greater than about 60% cause the film to become brittle and more paper-like rather than soft and flexible.

The textured dimensionally stable film of the present invention also has improved breathability properties due to the orientation of the film by intermesh gear (IMG) stretching of the film. When the film is stretched in the machine direction only, the preferred stretch is about 5 to 100%, and most preferably about 15 to 100%. It has been found that a film stretched at least 15% provides a MVTR of about 1000 to about 1500g/m²/day (grams per square meter per day).

The degree that the gears overlap or intermesh on the intermeshing gears, known as the "depth of engagement", can be varied to change the amount of orientation or stretching of the film. An increase in the depth of engagement of the intermeshing gears increases the breathability of the film. The distance between teeth on the intermeshing gears, known as "pitch", can also be varied to change the amount of orientation or stretch of the film. In practice it has been found that a distance of about 7.62 x 10⁻⁴ to about 5.08 x 10⁻⁴ m (30 to about 200 mils) between teeth·("pitch") and about 1.27 x 10⁻⁴ to about 7.62 x 10⁻⁴m (5 to about 200 mils) depth of engagement (DOE) on the IMG rolls can be used to produce a film having a MVTR of about 500 to about 5000 g/m²/day. A preferred range of pitch of about 1.27 x 10⁻³ to about 2.54 x 10⁻³ m (50 to about 100 mils) and a preferred depth of engagement of about 5.08 x 10⁻⁴ to about 1.9 x 10⁻⁵ m (20 to about 75 mils) provides a film having a MVTR of about 1500 to about 3000 g/m²/day.

The film formulation in combination with the orienting step of the present invention allows the film to have sufficient residual elongation so that the film can be further stretched. This further stretching of the film is often done by a customer during converting of the film into a disposable product. An important characteristic which allows for the additional stretching of the film is the residual elongation, or difference between the elongation-to-yield and the elongation-to-break of the film. The elongation is measured by a tensile tester as specified in ASTM D882. The difference between the percent of elongation-at-yield and the percent of elongation-at-break provides a "window of elongation" which is not found in other films. The film of the present invention has a residual elongation of greater than about 75% in the machine direction and greater than about 300% in the transverse direction, as measured by the difference between the percent of elongation-at-break and the percent of elongation-at-yield. It has been found that a film having a residual elongation of greater than about 150% in the machine direction (MD) and greater than about 500% in the transverse direction (TD) works particularly well. This increases the impact strength of the film.

The combination of film formulation and intermesh gear orientation also affects the impact strength of the film. The impact strength can be measured by dropping a dart onto the film as specified in ASTM D1709-80. When a film is stretched only in one direction there is less impact strength. Further, the higher level of stretch imparted to the film, the lower the impact strength. In the cast film embodiment, however, it is desired to stretch the film in the transverse direction more than in the machine direction due to the lack of TD direction orientation in cash films. It is also desirable to balance the machine direction orientation impacted to the film during manufacture with at least some amount of transverse direction orientation. In the blown film embodiment, the inherent transverse direction orientation may be such that transverse direction IMG orientation is unnecessary. In the formulation of the present invention, a higher percent of filler along with a highly orientable blend of polymers in the film provides breathability and allows for lower amounts of stretching.

The film of the present invention has an improved dimensional stability at elevated temperatures over prior art films. The oriented film is annealed to provide dimensional stability at elevated temperatures and heat resistance to the film. The annealing allows slight molecular relaxation of the stressed polymer chains of the polyolefins in the film formulation. The annealing step applies heat to the film at controlled tension to relieve the stress which resulted from the forming and orienting steps. Due to the relaxation which occurs in the annealing step there is less than about 15% shrinkage of the film after exposure to 76.67°C (170°F) air for 30 minutes. When the annealed film undergoes further processing steps, the film does not shrink when exposed to temperatures at or below the annealing temperature. The annealed film can withstand further heating such as when the film is subjected to converting processes and/or shipping environments. The film can be readily used in further converting steps such as when the hot glue is used to secure the microporous film to other components of a construction.

During the annealing step, tension between the annealing rolls is important to control. It is undesirable to have a positive draw between the annealing rolls. The positive draw stretches the film and undesirably reduces the dimensional stability at elevated temperatures. It is usually desired to have a negative draw as the film passes through the annealing rolls such that the first roll runs faster than the second roll. When the first roll runs faster than the second roll, the film speed slows somewhat and the film is allowed to relax. The relative speeds of the rolls determines the relaxation of the film. The second annealing roll speed is preferably about .90 to about 1.00 times the speed of the first annealing roll. However, too much relaxation will cause the micropores in the film to close. For example, if the second roll speed is about .85 to about .89 times the first annealing roll speed, then the micropores in the film will tend to close and reduce the breathability of the film. During the annealing step there is a minor increase in the thickness of the film due to the relaxation of the film.

As previously explained, the dimensional stability of the film is only increased to the temperature at which the film is annealed. Accordingly, it is necessary to balance the annealing temperatures against the softening point of the film formulation and the expected end use temperatures for the film.

The surface temperature of the annealing rolls should not be higher than the softening temperature of the film where it begins to stick to the annealing rolls. In addition, the annealing temperature is preferably higher than the temperatures that the film will experience either in further converting steps (hot glue application to secure film to parts of a disposable product) or in situations experienced by the film or disposable product (high temperatures while being shipped or stored). In most embodiments the film is annealed at about 82.2°C to 93.3°C (180°F to 200°F). The film is in contact with the annealing rolls long enough to allow the film to be sufficiently heated. It is important to heat the film to a desired temperature before the film relaxes between the rolls. In preferred embodiments, the residence time on each annealing roll is less than about 1 second.

The annealing relieves the stresses in the film, sets the film, prevents the film from shrinking, and prevents closing of the micropores in the film. The annealed film can be reheated and embossed without loss of any desired moisture vapor transmission rate, dimensional stability or elongation properties. If a film is oriented using an intermeshing gear orientation process and is embossed without an annealing step, the micropores will close and the breathability of the film will be impaired.

In certain embodiments, the film of the present invention can be embossed. The embossing step provides a desired texture to the oriented and annealed microporous film. The texturing provided by the embossing step improves the aesthetics and provides other physical advantages to the film. One especially useful advantage is that the embossing of the film reduces the contact surface area between any pressure sensitive adhesive and the microporous film such that less peeling force is needed to remove the adhesive strip from the film or to pull the film from an article to which it is attached.

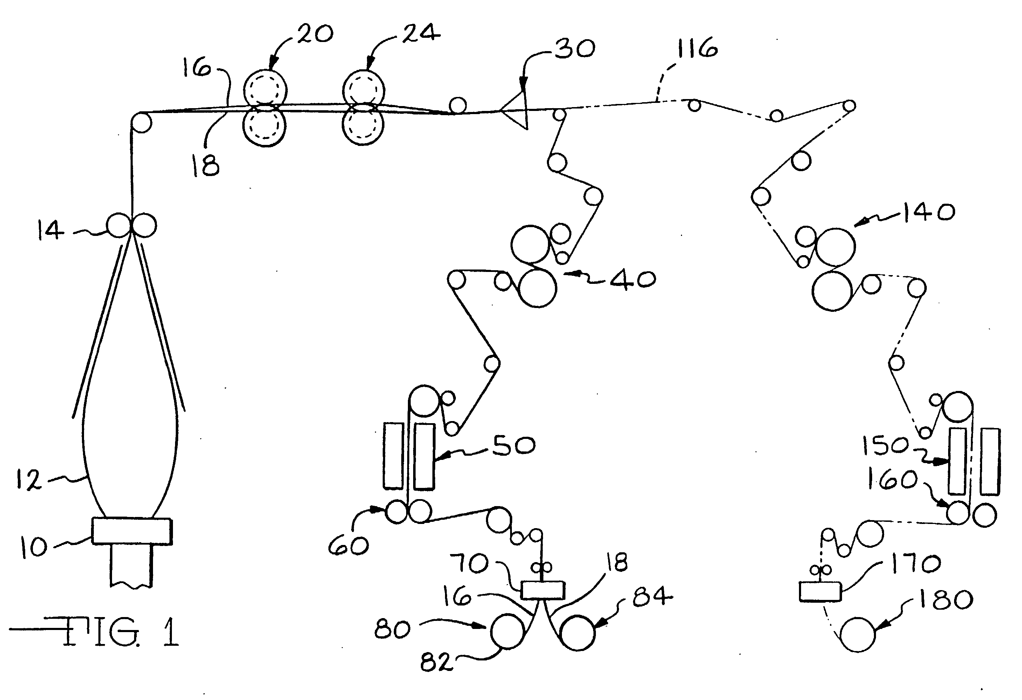

Fig. 1 is a schematic illustration of a system used to produce a soft texture dimensionally stable microporous film. In the embodiment shown in Fig. 1, a high filler content film formulation is extruded from a die 10 as a blown tubing 12. However, it should be understood that in other embodiments a cast extrusion method can be used to form a film. Various methods for forming blown and/or cast films can be practiced with the present invention. For ease of illustration, only the blown film method is shown in Fig. 1. The tubing 12 is collapsed by passing through a set of nip rolls 14. The tube 12 passes through a slitting apparatus 30 which slits the tubing 12 into two separate plies of film 16 and 18. The two plies 16 and 18 are biaxially oriented by passing through a first set of intermesh gear rolls 20 which stretch the film in the transverse direction. The two plies 16 and 18 then pass through a second set 24 of intermesh gear rolls which stretch the two plies in the machine direction. In other embodiments, it is contemplated that the plies can be uniaxially oriented by passing the tubing through either the machine direction intermesh gears or the transverse direction intermesh gears.

In the embodiment shown, the two plies of film 16 and 18 are separated after being oriented. The film 18 passes through a set of annealing rolls 40 that are at a temperature from about 82.2°C to about 93.3°C (180°F to about 200°F). The annealed film 18 passes adjacent a heating means 50 which reheats the annealed film 18 to a temperature from about 82.2°C to about 93.3°C (180°F to about 200°F). The reheated annealed film 18 can pass through a set of embossing rolls 60 which provides the annealed film 18 with an embossed pattern which gives softness and texture to the film 18. It is also within the contemplated scope of the present invention that the film 18 can pass from the annealing rolls 40 and pass through the embossing rolls without being subjected to further heat. It is desired that the film be at temperature below the melting temperatures but above the softening temperatures of the film formulation when the film is embossed.

In the embodiment shown, the embossed film 18 passes through a corona treatment means 70 which applies a high voltage to the surface of the film 18. It is to be understood, however, the film 18 may not be corona treated. The film 18 passes through winders 80 and is wound onto a roll 84 In the embodiment shown in Fig. 1, the film 16 also passes through an annealing means 40, reheating means 50, embossing means 60, corona treatment means 70 and is wound on a roll 82, in a manner as described for the film 18 above.

It is also within the contemplated scope of the present invention that the plies of film 16 and 18 can be separately oriented. In the embodiment shown in phantom in Fig. 1, the tubing 12 passes through the slitting apparatus 30 which slits the tubing into two separate plies of film 116 and 18. The film 116 is biaxially oriented by passing through a first set of intermeshing gear rolls 120 which stretch the film in the transverse direction. The film 116 then passes through a second set 124 of intermesh gear rolls which stretch the film 116 in the machine direction. The film 116 then passes through the annealing rolls 140, the heating means 150 and the embossing rolls 180, as described above.

The invention is further described in the examples below, although it should be understood that these examples are for the purposes of illustration, and are not intended to limit the scope of the invention.

### EXAMPLE 1

The relationship between the moisture vapor transmission rate and various formulations of the film in the present invention was evaluated. The balance between the high density polyethylene component and the linear low density polyethylene component in the film affects the moisture vapor transmission in the microporous films which have been formed by using intermeshing gear rolls to stretch the film in the machine direction. The following formulation was used in this example: 50%, by wt. calcium carbonate, X%, by wt. high density polyethylene, 46-X%, by wt. linear low density polyethylene, 2%, by wt. white concentrate in low density polyethylene; and 2%, by wt. fluorocarbon processing aid in low density polyethylene. All the films were produced at a 3.81 x 10⁻⁵m (1.5 mil) thickness and were machine direction oriented using intermeshing gears at an engagement depth of 1.27 x 10⁻³m (50 mils). The moisture vapor transmission rate results for these formulations is shown in Table 1 below. The MVTR test results in Table 1 were determined using ASTM E96-93 at 38°C and 75% relative humidity, using a sample cup having about 1.29 x 10⁻³ m² (2 in.²) open area.

**TABLE 1**

| LLDPE/HDPE Microporous Blends | |
|---|---|
| %HDPE | (g/m²-day) MVTR |
| 0 | 1279 |
| 6 | 1584 |
| 10 | 1768 |
| 12 | 2152 |

### EXAMPLE 2

Film samples of various formulations were tested to measure "glue burn through" performance. "Glue burn through" means that the film develops holes or melts in the area that the glue contacts during further converting. Glue burn through is an unacceptable characteristic in films used for disposable articles. Glue burn test results are reported on a scale of 0 to 3, with 0 being no bum through and 3 being massive burn through. These are measured by the observations of the operator conducting the test. The data shown in Table 2 below shows the glue bum through results.

**TABLE 2**

| Thickness | Base Polymer(s) | CaCO₃ Loading | MD/TD DOE | Annealer Temp. | Glue Burn Through Rating |
|---|---|---|---|---|---|
| 1.00 | LLDPE | 50% | 45/30 | N/A | 3 |
| 1.00 | LLDPE/10% HDPE | 50% | 45/30 | N/A | 1 |
| 1.00 | ULDPE | 50% | 45/30 | 200 | 0 |
| 1.00 | ULDPE/12% HDPE | 50% | 45/30 | N/A | 3 |

### EXAMPLE 3

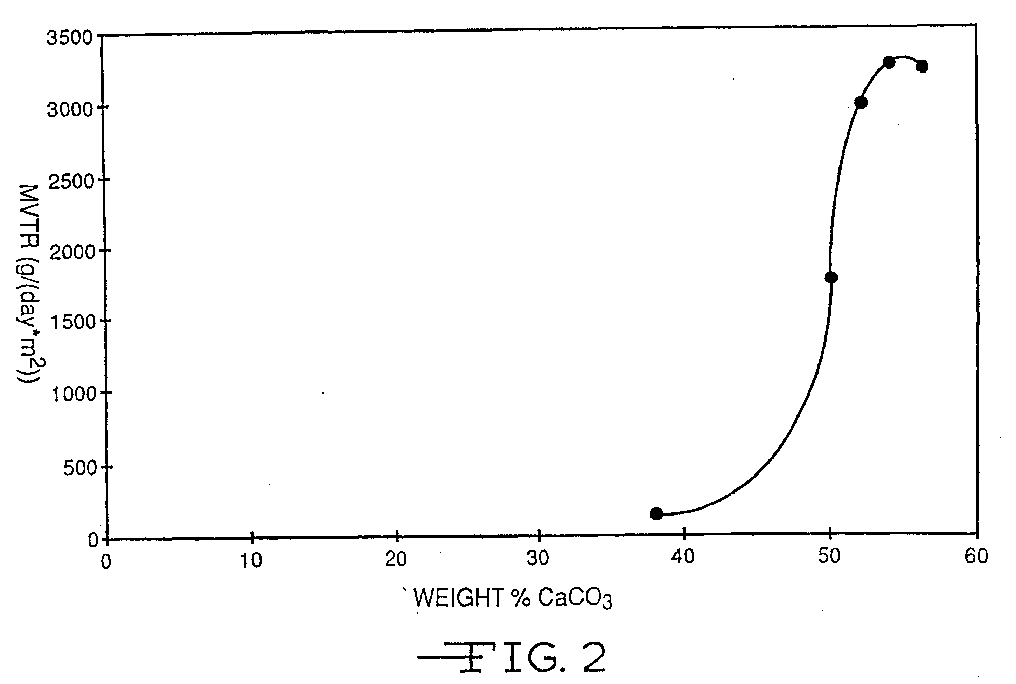

The relationship between amount of filler content in the film formulation and moisture vapor transmission rate was evaluated. Fig. 2 shows the moisture vapor transmission rate results of machine direction oriented films comprising X%, by wt. calcium carbonate filler, 86-X%, by wt linear low density polyethylene, 10%, by wt. high density polyethylene, 2%, by wt. white concentrate in low density polyethylene, and 2%, by wt. fluorocarbon processing aid in low density polyethylene. This formulation was used to produce a 3.81 x 10⁻⁵ m (1.5 mil) thick film which was machine direction oriented using intermeshing gear rolls at a 1.27 x 10⁻³ m (50 mil) depth of engagement. There is a significant increase in the moisture vapor transmission rate as the percent of filler approaches 50%. In preferred embodiments, the filler content ranges from about 45 to 60%. The most preferred amount of filler is about 50-54%. The moisture vapor transmission rate no longer exponentially increases when the percentage of calcium carbonate increases beyond about 54-55%.

### EXAMPLE 4

The effects of annealing on the physical properties of the intermeshing gear oriented microporous film were evaluated. Annealing of the films reduces the tendency of the microporous films to exhibit shrinkage without reducing breathability. Tests were conducted using a film containing 50% by wt., filler, 34.06%, by wt. LLDPE, 12%, by wt. HDPE, 1%, by wt. LDPE, 2%, by wt. titanium oxide concentrate, and .04%, by wt. fluoropolymer concentrate processing aid. The transverse direction and machine direction depths of engagement on the intermeshing gears rolls were held constant at 5.08 x 10⁻⁴ and 1.27 x 10⁻³ m (20 and 50 mils), respectively. The annealing rolls were held at a constant temperature of 82.2°C (180°F). The roll speed was measured at two levels, 13.11 and 35.05m (43 and 115 ft.) per minute.

Control films were produced by passing the films through the intermeshing gear rolls without annealing. The annealed films were passed through annealing rolls having a negative draw in order to minimize web tension and permit molecular relaxation to occur. Shrink tests were conducted at 71.1°C (160°F) unrestrained for five (5) minutes. Table 3 below shows the percent of shrink being approximately ½ to ¼ that of the controlled non-annealed film with no reduction in breathability. The MVTR values in Table 3 were obtained using ASTM 96-93 as modified in Example 1.

**TABLE 3**

| Physical Properties of Microporous Films | | | | |
|---|---|---|---|---|
| Annealer Roll Speed (m/min (ft/min)) | Control Sample | | Annealed Sample (82.2°C (180°F) Anneal) | |
| | Shrink | MVTR | Shrink | MVTR |
| 13.11 (43) | 24.3% | 1620 | 6.7% | 1760 |
| 35.05 (115) | 14.0% | 1590 | 7.3% | 1670 |

### EXAMPLE 5

The effect of intermesh gear roll orientation of a tubing rather than single plies of film was evaluated. The tubing does not block after intermeshing gear orientation and annealing. Rather, the tubing readily separates into single plies. The intermeshing gear roll orientation and annealing of a blown tubing produces two equivalent plies of film.

### EXAMPLE 6

The effects of embossing on an annealed MD IMG oriented microporous film was evaluated. An annealed MD IMGed microporous film was reheated and embossed using three embossing patterns.

The 1.40 mil precursor film was comprised of the following formulation: 54%, by wt. CaCO₃; 33.96%, by wt., LLDPE; 10%, by wt., HDPE; 0.67%, by wt., LDPE; 1.33%, by wt., titanium dioxide; and 0.04%, by wt., fluorocarbon. The films were extruded, stretched, and annealed. The annealed MD IMG oriented microporous films were reheated and embossed at a nip pressure of 6 607.5 Kg/m (370 lb/in). The pre-embossing roll temperature was 87.78°C (190°F), the engrave roll temperature was 51.67°C (125°F) and the cooling roll temperature was 35°C (95°F) for all samples. The line speed was 45m/min (150 ft/min) for all samples.

The three embossing patterns, macro hexagonal pattern (Mac), large diamond, and small diamond patterns, were embossed into an MD IMG oriented microporous film without significantly changing the MVTR or heat shrink values as shown in Table 4 below. The level of texture can be correlated with the differences in the low load thickness before and after embossing. As shown in Table 4, embossing enhanced the texture present after IMG orientation. The MVTR values in Table 4 were obtained using ASTM 96-93 as modified in Example 1.

**TABLE 4**

| | Embossing Pattern | | |
|---|---|---|---|
| | Mac | Small Diamond | Large Diamond |
| MVTR (g/m²-day) | 3345 | 3379 | 3327 |
| MD Heat Shrink (%) | 4.0 | 5.3 | 4.3 |
| Low Load Thickness After Embossing (m (mills)) | (3.82) 9.70 x 10⁻⁵ | (5.49) 1.39 x 10⁻⁵ | (3.52) 8.94 x 10⁻⁵ |
| Low Load Thickness Before Embossing (m (mils)) | (1.05) 2.67 x 10⁻⁵ | (0.92) 2.34 x 10⁻⁵ | (0.93) 2.36 x 10⁻⁵ |

### EXAMPLE 7

Table 5 below shows a comparison of the film of the present invention to other films currently being used as diaper backsheets. The key physical properties of breathable films for use in diaper backsheets are: breathability (MVTR), the residual elongation, dimensional stability at elevated temperatures during conversion and during shipping, impact strength while in use, and texture. The MVTR values in Table 5 were obtained using ASTM 96-93 as modified in Example 1.

The following samples were tested. Compar. Sample 1 is HTS-5 film sold by Tredegar Industries, a prior used diaper backsheet film. Compar. Sample 2 is a CPC 2 film sold by Tredegar Industries as a currently used diaper backsheet film. Compar. Sample 3 Exxon® Exxaire is a commercially available breathable film. Ex. A is an IMG oriented unannealed breathable film. Ex. B-1 and B-2 are IMG oriented annealed breathable films. Ex. C is an IMG oriented annealed and embossed breathable film. Examples A, B-1, B-2 and C comprise: 55%, by wt., CaCO₃; 31% by wt., LLDPE, 10% by wt., HDPE; 2%, by wt., white concentrate; and 2%, by wt. Viton® fluorocarbon concentrate. The film of the present invention has a preferred residual elongation of about 200% in the machine direction and about 300% in the transverse direction, as measured by the difference between the percent of elongation-at-break and the percent of elongation-at-yield.

While particular embodiments of the present invention have been illustrated and described, those skilled in the art will recognize that various changes and modifications can be made without departing from the spirit and scope of the invention. It is intended to cover, in the appended claims, all such modifications that are within the scope of this invention.

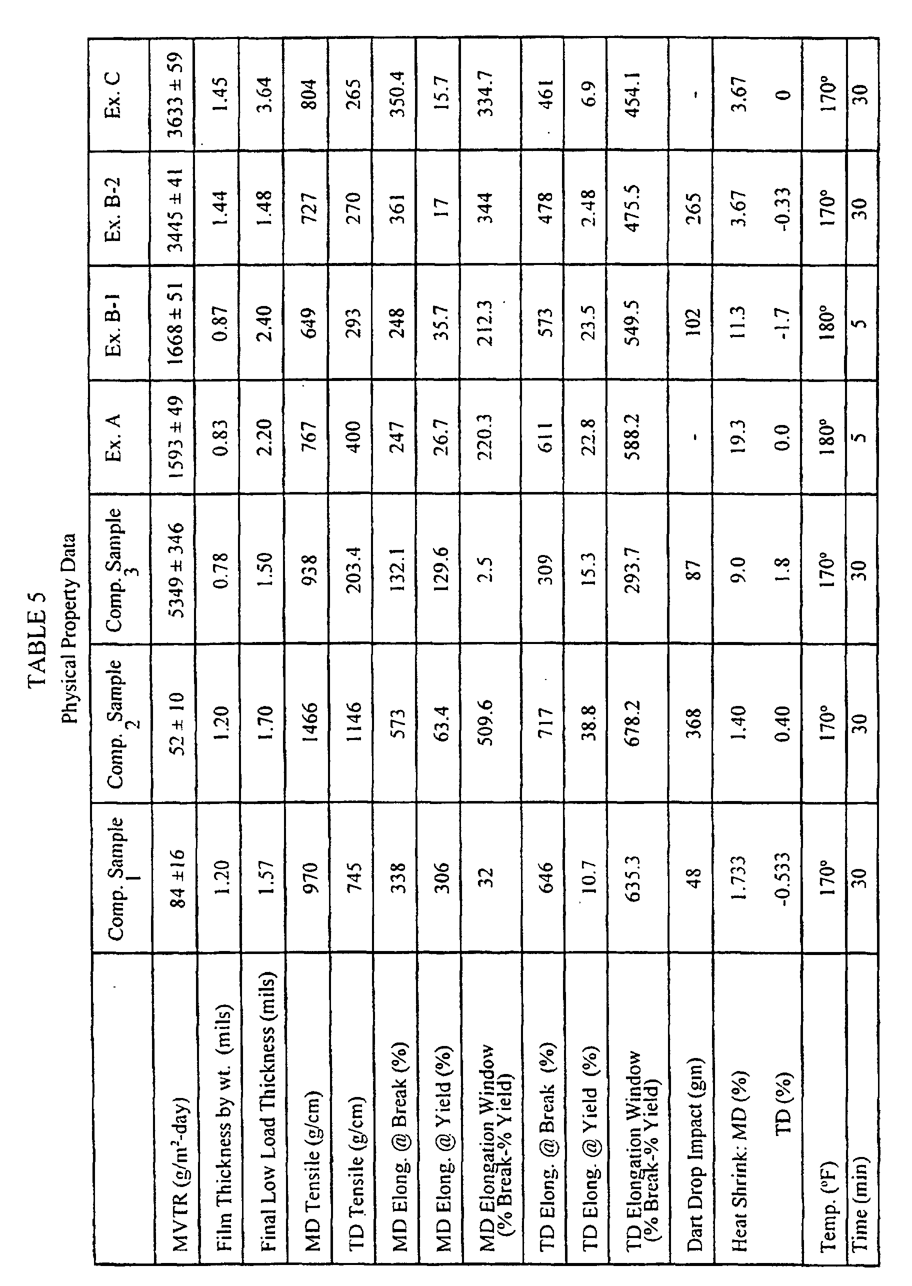

## Claims

1. A soft, textured, intermesh gear oriented microporous film that avoids shrinkage at temperatures up to 60°C (140°F) said film comprising 45 to 65%, by wt., filler; 25 to 55%, by wt., linear low density polyethylene; about 0 to about 20%, by wt., high density polyethylene ; 0 to 5%, by wt., low density polyethylene; about 0 to about 10%, by wt., pigmenting agent; and, about 0 to about 3%, by wt., processing aid.

2. The film of claim 1, wherein the film has a moisture vapor transmission rate of at least about 500 g/m²-day.

3. The film of claim 1, wherein the film has a percent of shrinkage less than 15% after exposure to 76.67°C (170°F) air for 30 minutes.

4. The film of claim 1, wherein the film has a residual elongation of greater than 75% machine direction and greater than 300% transverse direction as measured by the difference between the percent of elongation-at-break and the percent of elongation-at-yield.

5. The film of claim 1, wherein the film has a residual elongation of greater than 150% machine direction and greater than 400% transverse direction as measured by the difference between the percent of elongation-at-break and the percent of elongation-at-yield.

6. The film of claim 1, wherein the filler comprises calcium carbonate.

7. The film of claim 6, wherein the calcium carbonate filler is at least partially coated with a stearic acid or other stearate compound.

8. The film of claim 7, wherein the formulation comprises: 50 to 56% by wt., calcium carbonate filler; 30 to 35%, by wt., linear low density polyethylene; about 10 to about 12%, by wt., high density polyethylene; 0 to 50%, by wt., low density polyethylene; about 0 to about 2%, by wt., titanium dioxide; and, about 0 to about .05%, by wt., fluorocarbon processing aid.

9. The film of claim 8, wherein the formulation comprises 53 to 56%, by wt., calcium carbonate.

10. The film of claim 9, wherein the calcium carbonate has an average size of 0.5 to 5 microns.

11. The film of claim 1, wherein said film comprises 50%, by wt., calcium carbonate filler; 35%, by wt., LLDPE; 12%, by wt., HDPE; 1%, by wt., LDPE; 2%, by wt., titanium dioxide; and, 0.4%, by wt., fluorocarbon processing aid.

12. The film of claim 1, wherein said film comprises 56%, by wt., calcium carbonate filler, 30%, by wt., LLDPE; 10%, by wt., HDPE; 0.67% LOPE; 1.33%, by wt., titanium dioxide; and, 0.04%, by wt., fluorocarbon processing aid.

13. A method for forming a soft textured microporous film having a formulation comprising 45 to 65%, by wt., filler; 25 to 55%, by wt., linear low density polyethylene; about 0 to about 20%, by wt., high density polyethylene or other heat resistant polymer; 0 to 5%, by wt., low density polyethylene; about 0 to about 10%, by wt., pigmenting agent; and, about 0 to about 3%, by wt., fluorocarbon processing aid, which method comprises,
extruding the formulation as a film,
introducing the film into at least one set of
intermesh gear rolls to orient the film, and
passing the oriented film through an annealing means to anneal the film.

14. The method of claim 13, which further comprises reheating the annealed film and embossing the reheated film by passing the film through a nip of embossing rolls to impart an embossed pattern on the film.

15. The method of claim 13, in which the embossed film is subjected to a corona discharge treatment.

16. The method of claim 13, in which the film is extruded using a cast extrusion method.

17. The method of claim 13, in which the film is extruded using a blown film method.

18. The method of claim 17, in which the film is extruded as a bubble and the method includes collapsing the bubble to form a two-ply tubing and orienting both plies of the tubing together.

19. The method of claim 17, in which the film is extruded as a bubble and the method includes collapsing the bubble to form a two-ply tubing, separating the tubing into a first ply of film and a second ply of film, and orienting each ply separately.

20. The method of claim 13, in which the set of intermesh gear rolls orients the film in the machine direction.

21. The method of claim 20, further including a second set of intermesh gear rolls which orients the film in the transverse direction.

22. The method of claim 13, in which the set of intermesh gear rolls orient the film in the transverse direction.

23. The method of claim 13, in which the film is stretched 5% to 100% in the machine direction and/or the transverse direction.

24. The method of claim 13, in which the film is stretched 15% to 60% in the machine direction and/or the transverse direction.

25. The method of claim 13, in which the intermesh gears which orient the film have a plurality of intermeshing teeth spaced apart at a distance or pitch of 7.62 x 10⁻⁴ to 5.08 x 10⁻³m (30 to 200 mils) and the plurality of teeth intermesh or have a depth of engagement of 1.27 x 10⁻⁴ to 7.62 x 10⁻⁴m (5 to 200 mils).

26. The method of claim 25, in which the pitch ranges from 1.27 x 10⁻³ to 2.54 x 10⁻³m (50 to 100 mls) and the depth of engagement ranges from 5.08 x 10⁻⁴ to 1.9 x 10⁻³m (20 to 75 mls).

27. The method of claim 13, in which said film has a formulation comprising about 50%, by wt., calcium carbonate; about 35%, by wt., linear low density polyethylene; about 12%, by wt., high density polyethylene; about 1%, by wt., low density polyethylene; about 2%, by wt., titanium dioxide; and, about 0.04%, by wt., fluorocarbon processing aid, which method comprises,
reheating the annealed film, and
embossing the reheated film by passing the film through a nip of embossing rolls to impart an embossed pattern on the film.

28. The method of claim 13, in which said film has a formulation comprising 56%, by wt., calcium carbonate; 30%, by wt., linear low density polyethylene; 10%, by wt., high density polyethylene; 0.67%, by wt., low density polyethylene; 1.33%, by wt., titanium dioxide; and, 0.04%, by wt., fluorocarbon processing aid, which method comprises.
reheating the annealed film, and
embossing the reheated film by passing the film through a nip of embossing rolls to impart an embossed pattern on the film.

## Patentansprüche

1. Weiche, texturierte, mit einer Verzahnung orientierte, mikroporöse Folie, bei der ein Schrumpfen bei Temperaturen bis 60°C (140°F) vermieden wird, wobei die Folie 45 bis 65 Gew.-% Füllstoff, 25 bis 55 Gew.-% lineares Polyethylen geringer Dichte, etwa 0 bis etwa 20 Gew.-% Polyethylen hoher Dichte, 0 bis 5 Gew.-% Polyethylen geringer Dichte, etwa 0 bis etwa 10 Gew. - % Pigmentiermittel und etwa 0 bis etwa 3 Gew.-% Verarbeitungshilfsmittel enthält.

2. Folie nach Anspruch 1, wobei die Folie eine Wasserdampfdurchlässigkeitsrate von mindestens etwa 500g/m² pro Tag aufweist.

3. Folie nach Anspruch 1, wobei die Folie einen prozentualen Schrumpf von weniger als 15% aufweist, nachdem sie Luft mit 76,67°C (170°F) 30 Minuten lang ausgesetzt wurde.

4. Folie nach Anspruch 1, wobei die Folie eine bleibende Dehnung von mehr als 75% in Maschinenrichtung und von mehr als 300% in Querrichtung aufweist, bestimmt durch die Differenz zwischen der prozentualen Reißdehnung und der prozentualen Dehnung bei Streckgrenze.

5. Folie nach Anspruch 1, wobei die Folie eine bleibende Dehnung von mehr als 150% in Maschinenrichtung und von mehr als 400% in Querrichtung aufweist, bestimmt durch die Differenz zwischen der prozentualen Reißdehnung und der prozentualen Dehnung bei Streckgrenze.

6. Folie nach Anspruch 1, wobei der Füllstoff Calciumcarbonat umfasst.

7. Folie nach Anspruch 6, wobei der Calciumcarbonat-Füllstoff zumindest teilweise mit einer Stearinsäure oder einer anderen stearatverbindung beschichtet ist.

8. Folie nach Anspruch 7, wobei die Mischung enthält: 50 bis 56 Gew.-% Calciumcarbonat-Füllstoff, 30 bis 35 Gew.-% lineares Polyethylen geringer Dichte, etwa 10 bis etwa 12 Gew.-% Polyethylen hoher Dichte, 0 bis 50 Gew.-% Polyethylen geringer Dichte, etwa 0 bis etwa 2 Gew.-% Titandioxid und etwa 0 bis etwa 0,05 Gew.-% Fluorcarbon-Verarbeitungshilfsmittel.

9. Folie nach Anspruch 8, wobei die Mischung 53 bis 56 Gew.-% Calciumcarbonat enthält.

10. Folie nach Anspruch 9, wobei das Calciumcarbonat eine durchschnittliche Größe von 0,5 bis 5 Mikron hat.

11. Folie nach Anspruch 1, wobei die Folie 50 Gew.-% Caiciumcarbonat-Füllstoff, 35 Gew.-% LLDPE, 12 Gew.-% HDPE, 1 Gew.-% LDPE, 2 Gew.-% Titandioxid und 0,4 Gew.-% Fluorcarbon-Verarbeitungshilfsmittel enthält.

12. Folie nach Anspruch 1, wobei die Folie 56 Gew.-% Caiciumcarbonat-Füllstoff, 30 Gew.-% LLDPE, 10 Gew.-% HDPE, 0,67% LDPE, 1,33 Gew.-% Titandioxid und 0,04 Gew.-% Fluorcarbon-Verarbeitungshilfsmittel enthält.

13. Verfahren zur Herstellung einer weichen, texturierten, mikroporösen Folie mit einer Mischung, die 45 bis 65 Gew.-% Füllstoff, 25 bis 55 Gew.-% lineares Polyethylen geringer Dichte, etwa 0 bis etwa 20 Gew.-% Polyethylen hoher Dichte oder anderes hitzebeständiges Polymer, 0 bis 5 Gew.-% Polyethylen geringer Dichte, etwa 0 bis etwa 10 Gew.-% Pigmentiermittel und etwa 0 bis etwa 3 Gew.-% Fluorcarbon-Verarbeitungshilfsmittel enthält, wobei das Verfahren umfasst:
Extrudieren der Mischung als eine Folie,
Einführen der Folie in mindestens einen Satz von ineinander verzahnten Walzen, um die Folie zu orientieren, und
Leiten der orientierten Folie durch eine Entspannungseinrichtung, um die Folie spannungsfrei zu machen.

14. Verfahren nach Anspruch 13, das außerdem das erneute Erwärmen der spannungsfrei gemachten Folie und das Prägen der erneut erwärmten Folie umfasst, indem die Folie durch einen Spalt zwischen Prägewalzen geleitet wird, um auf die Folie ein geprägtes Muster aufzubringen.

15. Verfahren nach Anspruch 13, bei dem die geprägte Folie einer Korona-Entladungsbehandlung ausgesetzt wird.

16. Verfahren nach Anspruch 13, bei dem die Folie unter Verwendung eines Formextrusionsverfahrens extrudiert wird.

17. Verfahren nach Anspruch 13, bei dem die Folie unter verwendung eines Blasfolienverfahrens extrudiert wird.

18. Verfahren nach Anspruch 17, bei dem die Folie als eine Blase extrudiert wird und das Verfahren das zusammenfallen der Blase, um einen Doppelschicht-Schlauch zu bilden, und das gemeinsame Orientieren beider Schichten des Schlauchs umfasst.

19. Verfahren nach Anspruch 17, bei dem die Folie als eine Blase extrudiert wird und das Verfahren das Zusammenfallen der Blase, um einen Doppelschicht-Schlauch zu bilden, das Trennen des Schlauchs in eine erste Folienschicht und eine zweite Folienschicht und das getrennte Orientieren jeder Schicht umfasst.

20. Verfahren nach Anspruch 13, bei dem der Satz ineinanderverzahnter Walzen die Folie in Maschinenrichtung orientiert.

21. Verfahren nach Anspruch 20, außerdem mit einem zweiten Satz ineinanderverzahnter Walzen, die die Folie in der Querrichtung orientieren.

22. Verfahren nach Anspruch 13, bei dem der Satz ineinanderverzahnter Walzen die Folie in Querrichtung orientiert.

23. Verfahren nach Anspruch 13, bei dem die Folie um 5% bis 100% in Maschinenrichtung und/oder Querrichtung gereckt wird.

24. Verfahren nach Anspruch 13, bei dem die Folie um 15% bis 60% in Maschinenrichtung und/oder in Querrichtung gereckt wird.

25. Verfahren nach Anspruch 13, bei dem die Verzahnung, durch die die Folie orientiert wird, eine Vielzahl von ineinanderverzahnten Zähnen aufweist, die mit einem Abstand bzw. Zwischenraum von 7,62 x 10⁻⁴ bis 5,08 x 10⁻³ in (30 bis 200 Mil) angeordnet sind, und die Vielzahl von Zähnen ineinandergreifen oder eine Eingriffstiefe von 1,27 x 10⁻⁴ bis 7,62 x 10⁻⁴ m (5 bis 200 Mil) haben.

26. Verfahren nach Anspruch 25, bei dem der Zwischenraum von 1,27 x 10⁻³ bis 2,54 x 10⁻³ m (50 bis 100 Mil) reicht und die Eingriffstiefe 5,08 x 10⁻⁴ bis 1,9 x 10⁻³ m (20 bis 75 Mil) reicht.

27. Verfahren nach Anspruch 13, bei dem die Folie eine Mischung hat, die etwa 50 Gew.-% Calciumcarbonat, etwa 35 Gew.-% lineares Polyethylen geringer Dichte, etwa 12 Gew.-% Polyethylen hoher Dichte, etwa 1 Gew.-% Polyethylen geringer Dichte, etwa 2 Gew.-% Titandioxid und etwa 0,04 Gew.-% Fluorcarbon-Verarbeitungshilfsmittel enthält, wobei das Verfahren umfasst:
erneutes Erwärmen der spannungsfrei gemachten Folie und
Prägen der erneut erwärmten Folie, indem die Folie durch einen Spalt zwischen Prägewalzen geleitet wird, um auf die Folie ein geprägtes Muster aufzubringen.

28. Verfahren nach Anspruch 13, bei dem die Folie eine Mischung hat, die 56 Gew.-% Calciumcarbonat, 30 Gew.-% lineares Polyethylen geringer Dichte, 10 Gew. - % Polyethylen hoher Dichte, 0,67 Gew.-% Polyethylen geringer Dichte, 1,33 Gew.-% Titandioxid und 0,04 Gew.-% Fluorcarbon-Verarbeitungshilfsmittel enthält, wobei das verfahren umfasst:
erneutes Erwärmen der spannungsfrei gemachten Folie und
Prägen der erneut erwärmten Folie, indem die Folie durch einen Spalt zwischen Prägewalzen geleitet wird, um auf die Folie ein geprägtes Muster aufzubringen.

## Revendications

1. Film microporeux orienté par roues engrenées, texturé et souple, qui évite le rétrécissement à des températures allant jusqu'à 60°C (140°F), ledit film comprenant de 45 à 65 % en poids de charge ; de 25 à 55 % en poids de polyéthylène basse densité linéaire ; d'environ 0 à environ 20 % en poids de polyéthylène haute densité ; de 0 à 5 % en poids de polyéthylène basse densité ; d'environ 0 à environ 10 % en poids d'agent de pigmentation ; et d'environ 0 à environ 3 % en poids d'auxiliaire de mise en oeuvre.

2. Film selon la revendication 1, dans lequel le film a une vitesse de transmission de vapeur d'humidité d'au moins environ 500 g/m²-jour.

3. Film selon la revendication 1, dans lequel le film a un pourcentage de rétrécissement inférieur à 15 % après exposition à de l'air à 76,67°C (170°F) pendant 30 minutes.

4. Film selon la revendication 1, dans lequel le film a un allongement résiduel supérieur à 75 % dans la direction de la machine et supérieur à 300 % dans la direction transversale, tel que mesuré par la différence entre le pourcentage d'allongement à la rupture et le pourcentage d'allongement à la limite élastique.

5. Film selon la revendication 1, dans lequel le film a un allongement résidu supérieur à 150 % dans la direction de la machine et supérieur à 400 % dans la direction transversale, tel que mesuré par la différence entre le pourcentage d'allongement à la rupture et le pourcentage d'allongement à la limite élastique.

6. Film selon la revendication 1, dans lequel la charge comprend du carbonate de calcium.

7. Film selon la revendication 6, dans lequel la charge de carbonate de calcium est au moins partiellement enrobée d'un acide stéarique ou d'un autre composé stéarate.

8. Film selon la revendication 7, dans lequel la formulation comprend : de 50 à 56 % en poids de charge de carbonate de calcium ; de 30 à 35 % en poids de polyéthylène basse densité linéaire ; d'environ 10 à environ 12 % en poids de polyéthylène haute densité ; de 0 à 50 % en poids de polyéthylène basse densité ; d'environ 0 à environ 2 % en poids de dioxyde de titane ; et d'environ 0 à environ 0,05 % en poids d'auxiliaire de mise en oeuvre fluorocarboné.

9. Film selon la revendication 8, dans lequel la formulation comprend de 53 à 56 % en poids de carbonate de calcium.

10. Film selon la revendication 9, dans lequel le carbonate de calcium a une taille moyenne de 0,5 à 5 µm.

11. Film selon la revendication 1, dans lequel ledit film comprend 50 % en poids de charge de carbonate de calcium ; 35 % en poids de LLDPE ; 12 % en poids de HDPE ; 1 % en poids de LDPE ; 2 % en poids de dioxyde de titane ; et 0,4 % en poids d'auxiliaire de mise en oeuvre fluorocarboné.

12. Film selon la revendication 1, dans lequel ledit film comprend 56 % en poids de charge de carbonate de calcium ; 30 % en poids de LLDPE ; 10 % en poids de HDPE ; 0,67 % de LDPE ; 1,33 % en poids de dioxyde de titane ; et 0,04 % en poids d'auxiliaire de mise en oeuvre fluorocarboné.

13. Procédé pour former un film microporeux texturé souple ayant une formulation comprenant de 45 à 65 % en poids de charge ; de 25 à 55 % en poids de polyéthylène basse densité linéaire ; d'environ 0 à environ 20 % en poids de polyéthylène haute densité ou d'un autre polymère thermorésistant ; de 0 à 5 % en poids de polyéthylène basse densité ; d'environ 0 à environ 10 % en poids d'agent de pigmentation ; et d'environ 0 à environ 3 % en poids d'auxiliaire de mise en oeuvre fluorocarboné, lequel procédé comprend :
l'extrusion de la formulation sous la forme d'un film,
l'introduction du film dans au moins un ensemble de roues dentées engrenées pour orienter le film,
et
le passage du film orienté par un recuit signifie recuire le film.

14. Procédé selon la revendication 13, qui comprend en outre le réchauffage du film recuit et l'emboutissage du film réchauffé par passage du film au travers d'une ligne de contact de deux cylindres d'emboutissage pour réaliser un motif embouti sur le film.

15. Procédé selon la revendication 13, dans lequel le film embouti est soumis à un traitement par décharge couronne.

16. Procédé selon la revendication 13, dans lequel le film est extrudé au moyen d'un procédé d'extrusion par coulée.

17. Procédé selon la revendication 13, dans lequel le film est extrudé au moyen d'un procédé de film soufflé.

18. Procédé selon la revendication 17, dans lequel le film est extrudé sous la forme d'une bulle et le procédé comprend l'affaissement de la bulle pour former un tube à deux plis et l'orientation commune des deux plis du tube.

19. Procédé selon la revendication 17, dans lequel le film est extrudé sous la forme d'une bulle et le procédé comprend l'affaissement de la bulle pour former un tube à deux plis, la séparation du tube en un premier pli de film et un deuxième pli de film, et l'orientation de chaque pli séparément.

20. Procédé selon la revendication 13, dans lequel l'ensemble de roues dentées engrenées oriente le film dans le sens machine.

21. Procédé selon la revendication 20, comprend en outre un deuxième ensemble de roues dentées engrenées qui oriente le film dans le sens transversal.

22. Procédé selon la revendication 13, dans lequel l'ensemble de roues dentées engrenées oriente le film dans le sens transversal.

23. Procédé selon la revendication 13, dans lequel le film est étiré de 5 à 100 % dans le sens machine et/ou dans le sens transversal.

24. Procédé selon la revendication 13, dans lequel le film est étiré de 15 à 60 % dans le sens machine et/ou dans le sens transversal.

25. Procédé selon la revendication 13, dans lequel les roues engrenées qui orientent le film ont une pluralité de dents engrenées espacées entre elles d'une distance ou d'un pas de 7,62 x 10⁻⁴ à 5,08 x 10⁻³ m (30 à 200 mils) et la pluralité de dents s'engrènent ou ont une profondeur de prise de 1,27 x 10⁻⁴ à 7,62 x 1à⁻⁴ m (5 à 200 mils).

26. Procédé selon la revendication 25, dans lequel le pas est situé dans la plage allant de 1,27 x 10⁻³ à 2,54 x 10⁻³ m (50 à 100 mils) et la profondeur de prise est située dans la plage allant de 5,08 x 10⁻⁴ à 1,9 x 10⁻³ m (20 à 75 mils).

27. Procédé selon la revendication 13, dans lequel ledit film a une formulation comprenant environ 50 % en poids de carbonate de calcium ; environ 35 % en poids de polyéthylène basse densité linéaire ; environ 12 % en poids de polyéthylène haute densité ; environ 1 % en poids de polyéthylène basse densité ; environ 2 % en poids de dioxyde de titane ; et environ 0,04 % en poids d'auxiliaire de mise en oeuvre fluorocarboné, lequel procédé comprend :
le réchauffage du film recuit, et
l'emboutissage du film réchauffé par passage du film au travers d'une ligne de contact de deux cylindres d'emboutissage pour réaliser un motif embouti sur le film.

28. Procédé selon la revendication 13, dans lequel ledit film a une formulation comprenant 56 % en poids de carbonate de calcium ; 30 % en poids de polyéthylène basse densité linéaire ; 10 % en poids de polyéthylène haute densité; 0,67 % en poids de polyéthylène basse densité ; 1,33 % en poids de dioxyde de titane ; et 0,04 % en poids d'auxiliaire de mise en oeuvre fluorocarboné, lequel procédé comprend ;
le réchauffage du film recuit, et
l'emboutissage du film réchauffé par passage du film au travers d'une ligne de contact de deux cylindres d'emboutissage pour réaliser un motif embouti sur le film.
